# EUROPÄISCHE PATENTANMELDUNG

(11) **EP 4 796 208 A1**
(43) Veröffentlichungstag der Anmeldung: **26.08.2026**
(21) Anmeldenummer: 26159002.0
(22) Anmeldetag: 17.02.2026
(51) Int. Cl.: B01D 63/02, A61M 1/16

(54) **MEDIZINISCHE FILTERVORRICHTUNG UND EXTRAKORPORALE BLUTBEHANDLUNGSVORRICHTUNG MIT EINER MEDIZINISCHEN FILTERVORRICHTUNG**

(30) Priorität: 25.02.2025 DE 102025106988
(71) Anmelder: B. Braun Avitum AG, 34212 Melsungen (DE)
(72) Erfinder: KAESTNER, Falk, 01900 Bretnig-Hauswalde (DE)
(74) Vertreter: Winter, Brandl - Partnerschaft mbB

(57) **Zusammenfassung**

Die Offenbarung betrifft eine medizinische Filtervorrichtung (10), vorzugsweise Dialysator oder Ultrafilter, umfassend ein Gehäuse (11), eine Vielzahl von Hohlfasern (12), welche ein Faserbündel (13) bilden, das in dem Gehäuse (11) angeordnet ist, zumindest einen Anschluss (14), welcher an dem Gehäuse (11) angeordnet ist und welcher vorgesehen ist, ein Fluid, vorzugsweise Dialysierflüssigkeit, in das Gehäuse (11), insbesondere in einen Zwischenraum (15) zwischen der Vielzahl von Hohlfasern (12), einströmen zu lassen, einen zylindrischen Hohlkörper (16), insbesondere Diffusorring, der innerhalb des Gehäuses (11) derart angeordnet ist, dass sich ausgehend von dem zumindest einen Anschluss (14) ein Ringkanal (17) zwischen dem zylindrischen Hohlkörper (16) und dem Gehäuse (11) ausbildet, wobei der zylindrische Hohlkörper (16) das Faserbündel (13) umfasst und dabei zentrisch in dem Gehäuse (11) anordnet, wobei der zylindrische Hohlkörper (16) an seiner Mantelfläche (18) zumindest eine Öffnung (19) aufweist, die eine Querschnittsfläche (20) aufweist, wobei die zumindest eine Öffnung (19) durchgängig ist, wobei sich die Querschnittsfläche (20) zumindest teilweise in radialer Richtung von außen nach innen vergrößert, so dass sich eine Strömung des Fluids beim Durchströmen der zumindest einen Öffnung (19) verlangsamt. Die Offenbarung betrifft weiter eine extrakorporale Blutbehandlungsvorrichtung (30) mit einer solchen medizinischen Filtervorrichtung (10).

## Beschreibung

### Technisches Gebiet

Die vorliegende Offenbarung betrifft eine medizinische Filtervorrichtung sowie eine extrakorporale Blutbehandlungsvorrichtung mit einer medizinischen Filtervorrichtung.

### Technischer Hintergrund

Medizinische Filter sind aus dem Stand der Technik grundsätzlich bekannt. Die Filter werden unter anderem zur Reinigung von Blut im Rahmen einer Dialyse, das heißt als Dialysator, eingesetzt oder zur Filterung von Wasser, von Permeat, von Dialysierflüssigkeit oder von Substitutionsflüssigkeit. Die Filter können dabei Hohlfasern umfassen, durch die - im Falle eines Dialysators - das Blut fließt. Die Hohlfasern können dabei von einem Fluid umströmt werden. Durch Diffusions- und/oder Konvektionsprozesse über die Hohlfaserwand kann so das Blut gereinigt werden. Die Hohlfasern sind grundsätzlich fragil. Eine hohe Strömungsgeschwindigkeit des die Hohlfasern umströmenden Fluids kann die Hohlfasern beschädigen. Weiterhin ist man bestrebt, eine möglichst effiziente und gleichmäßige Filterung des Bluts zu erhalten. Diese benötigt eine möglichst gleichmäßige Umströmung der Hohlfasern.

In diesem Zusammenhang hat sich herausgestellt, dass ein Bedarf besteht, eine verbesserte medizinische Filtervorrichtung bereitzustellen.

### Zusammenfassung der vorliegenden Offenbarung

Es ist daher die Aufgabe der vorliegenden Offenbarung, die Nachteile aus dem Stand der Technik zu vermeiden oder wenigstens zu verringern und insbesondere eine verbesserte medizinische Filtervorrichtung bereitzustellen. Insbesondere ist es Aufgabe der vorliegenden Offenbarung, eine medizinische Filtervorrichtung bereitzustellen, welche eine Beschädigung der Hohlfasern vermindert bzw. möglichst vermeidet und welche eine gleichmäßige Umströmung der Hohlfasern ermöglicht.

Die Aufgabe der vorliegenden Offenbarung wird durch eine medizinische Filtervorrichtung mit den Merkmalen des Patentanspruchs 1 sowie durch eine extrakorporale Blutbehandlungsvorrichtung mit den Merkmalen des nebengeordneten Patentanspruchs 13 gelöst. Vorteilhafte Ausgestaltungen sind Gegenstand der Unteransprüche und/oder werden nachfolgend erläutert.

Ein erster Aspekt der vorliegenden Offenbarung betrifft eine medizinische Filtervorrichtung, vorzugsweise Dialysator oder Ultrafilter, umfassend: ein Gehäuse; eine Vielzahl von Hohlfasern, welche ein Faserbündel bilden, das in dem Gehäuse angeordnet ist; zumindest einen Anschluss, welcher an dem Gehäuse angeordnet ist und welcher vorgesehen ist, ein Fluid, vorzugsweise Dialysierflüssigkeit oder Substitutionsflüssigkeit, in das Gehäuse, insbesondere in einen Zwischenraum zwischen der Vielzahl von Hohlfasern, einströmen zu lassen; einen zylindrischen Hohlkörper, insbesondere Diffusorring, der innerhalb des Gehäuses derart angeordnet ist, dass sich ausgehend von dem zumindest einen Anschluss ein Ringkanal zwischen dem zylindrischen Hohlkörper und dem Gehäuse ausbildet; wobei der zylindrische Hohlkörper das Faserbündel umfasst und dabei zentrisch in dem Gehäuse anordnet; wobei der zylindrische Hohlkörper an seiner Mantelfläche zumindest eine Öffnung aufweist, die eine Querschnittsfläche aufweist; wobei die zumindest eine Öffnung durchgängig ist; und wobei sich die Querschnittsfläche zumindest teilweise in radialer Richtung von außen nach innen vergrößert, so dass sich eine Strömung des Fluids beim Durchströmen der zumindest einen Öffnung verlangsamt.

Der Begriff Dialysator meint vorliegend insbesondere einen Filter, der eingerichtet ist, Blut eines Patienten zu filtern, indem Abfallstoffe und/oder Giftstoffe aus dem Blut entfernt werden. Dabei fließen Blut und Dialysierflüssigkeit getrennt durch eine Membran (vorliegend Hohlfaser) aneinander vorbei. Die Abfallstoffe und/oder Giftstoffe diffundieren aufgrund eines Konzentrationsgefälles aus dem Blut in die Dialysierflüssigkeit. Frische Dialysierflüssigkeit gelangt in den Dialysator bevorzugt über den angesprochenen zumindest einen Anschluss. Verbrauchte Dialysierflüssigkeit bzw. Dialysat wird bevorzugt über einen weiteren Anschluss aus dem Dialysator abgeführt.

Der Begriff Ultrafilter meint vorliegend ein Filterelement, das eingerichtet ist, eine Flüssigkeit von Partikeln, Pilzen, Endotoxinen oder Ähnlichem zu trennen, indem die Flüssigkeit durch die Membran (vorliegend Hohlfaser) gefiltert wird, insbesondere von dem Zwischenraum zwischen der Vielzahl von Hohlfasern über Poren in das Innere der Hohlfasern gelangt, während die Partikel, Pilze, Endotoxine oder Ähnliches in dem Zwischenraum zwischen der Vielzahl von Hohlfasern verbleiben.

Der Begriff Gehäuse meint eine strukturelle Komponente, die das Faserbündel und den zylindrischen Hohlkörper umgibt und einen Strömungsbereich für das Fluid bereitstellt. Das Gehäuse kann einteilig oder mehrteilig ausgeführt sein. Das Gehäuse kann als Material Kunststoff umfassen. Das Gehäuse kann als Material Metall umfassen. Das Gehäuse kann eine zylindrische Form umfassen. Das Gehäuse kann einen oder mehrere Anschlüsse für eine Fluid und/oder eine zu filternde Flüssigkeit bzw. Ausgangsflüssigkeit, im Falle eines Dialysators beispielsweise als Anschlüsse einen Dialysierflüssigkeitseinlass, einen Dialysierflüssigkeitsauslass, einen Bluteinlass und einen Blutauslass, umfassen.

Der Begriff Hohlfaser meint eine Röhre, mit einer semipermeablen Wand (i. e. Membran). Eine zu filternde Flüssigkeit, im Falle eines Dialysators beispielsweise Blut, kann durch die Hohlfaser(n) hindurchfließen. Beispielsweise fließt das Blut durch die Hohlfaser(n) hindurch und wird von Abfallstoffen gereinigt, die durch die Wand der Hohlfaser(n) diffundieren. Alternativ, im Falle eines Ultrafilters, kann Wasser, Permeat, Dialysierflüssigkeit oder Substitutionsflüssigkeit durch die Wand bzw. Wände der Hohlfaser(n) hindurchfließen, insbesondere über die Poren der Wand bzw. Wände durchtreten. Dabei werden beispielsweise Bakterien, Partikel, Endotoxine, Pilze oder Ähnliches, die größer als die Poren sind, zurückgehalten. Auf diese Weise kann das Wasser/ das Permeat/ die Dialysierflüssigkeit/ die Substitutionsflüssigkeit gefiltert werden.

Der Begriff Faserbündel meint insbesondere eine Vielzahl an Hohlfasern. Das Faserbündel kann vorzugsweise über das Gehäuse mittels Polyurethan an zwei gegenüberliegenden Endbereichen eingegossen sein. Dadurch kann eine Fixierung der einzelnen Hohlfasern innerhalb des Gehäuses ermöglicht werden. Oberhalb bzw. unterhalb dieser Endbereiche kann vorzugsweise eine Schnittstelle/ ein Anschluss für einen Einlass bzw. einen Auslass einer zu filternden Flüssigkeit, beispielsweise Blut, angeordnet sein.

Der Begriff Anschluss meint vorliegend eine Schnittstelle zum Einlass oder zum Auslass eines Fluids.

Der Begriff zylindrischer Hohlkörper meint vorliegend ein strukturelles Bauteil, das eingerichtet ist, das Faserbündel zu umfassen und eine Strömung durch seine Wand zu verlangsamen. Der zylindrische Hohlkörper kann einteilig oder mehrteilig sein. Der zylindrische Hohlkörper kann als Material Kunststoff umfassen. Der zylindrische Hohlkörper kann als Material Metall umfassen. Der zylindrische Hohlkörper kann beispielsweise formschlüssig über Eingießen mittels Polyurethan innerhalb des Gehäuse angebunden sein. Der zylindrische Hohlkörper kann beispielsweise über eine Steckverbindung in dem Gehäuse angeordnet sein. Der zylindrische Hohlkörper kann eine Vielzahl an Öffnungen über seine Mantelfläche verteilt aufweisen, die in beliebigen Mustern verteilt angeordnet sein können. Die Öffnungen können sich in Form und/oder Dimension unterscheiden. Die Querschnittsfläche der Öffnung kann vorzugsweise von außen nach innen in radialer Richtung zunehmen.

Der Offenbarung liegt folgende Erkenntnis zugrunde: Filter, insbesondere Dialysatoren, werden nach dem Faserlecktest (Durchströmen der Fasern mit Wasser) mit Heißluft getrocknet. Um schneller zu trocknen, wird nicht nur das Innere der Hohlfasern sondern auch der Zwischenraum zwischen den Hohlfasern mit Heißluft durchströmt. Insbesondere gelangt Heißluft über den zumindest einen Anschluss, insbesondere Dialysierflüssigkeitsanschluss, in den Zwischenraum zwischen den Hohlfasern. Besonders im Bereich des zumindest einen Anschlusses entstehen dabei starke Turbulenzen, wodurch einzelne Hohlfasern in Schwingung versetzt werden. Sind die Schwingungen zu stark, können die Hohlfasern brechen. Im Stand der Technik wurden üblicherweise Prallplatten, die das direkte Auftreffen der Heißluft bzw. des Dialysierflüssigkeitsstroms auf das Faserbündel verhindern oder Prallringe, die den Dialysierflüssigkeitsstrom besser auf den Umfang des Faserbündels verteilen, verwendet. Sowohl Prallplatte als auch Prallring reduzieren die turbulente Strömung selbst nicht, sondern entlasten lediglich die Fasern im Bereich des Anschlusses dadurch, dass sie die turbulente Strömung auf den Umfang des Faserbündels verteilen. Die Offenbarung schlägt zur Lösung dieser Probleme einen zylindrischen Hohlkörper, insbesondere Diffusorring, vor, der Öffnungen mit sich vergrößernden Querschnittsflächen aufweist. Der zylindrische Hohlkörper kann dadurch vorteilhaft die Strömung gleichmäßig um den Umfang des Faserbündels verteilen, die Turbulenzen durch die Verlangsamung der Strömung reduzieren, sowie das Faserbündel in dem Gehäuse der Filtervorrichtung zentrieren. Dadurch kann sowohl eine schnellere Bereitstellung der Filtervorrichtung nach dem Faserlecktest ermöglicht werden als auch eine effizientere Filterung im Betrieb, als auch kann eine Beschädigung der Fasern sowohl während der Herstellung der Filtervorrichtung als auch während des Betriebs der Filtervorrichtung, auf verbesserte Weise vermieden werden.

In anderen Worten ausgedrückt, schützt der zylindrische Hohlkörper, welcher insbesondere als Diffusorring ausgebildet ist, die Fasern vor einer stark turbulenten dialysierflüssigkeitsseitigen Strömung und/oder vor einem stark turbulenten Heißluftstrom. Über eine entsprechende Verteilung der Öffnungen, insbesondere Diffusoröffnungen, wird eine besonders gleichmäßige Strömung über den Umfang des Faserbündels erreicht. Im Herstellprozess kann eine schnellere Trocknung mit Heißluft erfolgen. Eine besonders gleichmäßige Anströmung macht die Reduzierung der Faserwandstarke und damit auch mehr Filterleistung auf kleinerem Raum möglich.

Gemäß einer bevorzugten Ausführungsform kann die zumindest eine Öffnung eine Vielzahl an Öffnungen umfassen, die über einen Umfang des zylindrischen Hohlkörpers verteilt sind.

Die Öffnungen können identisch sein. Die Öffnungen können sich unterscheiden. Die Öffnungen können gleichmäßig über den Umfang verteilt sein. Die Öffnungen können unregelmäßig über den Umfang verteilt sein.

Auf diese Weise kann vorteilhaft eine bessere Umströmung des Faserbündels bei gleichzeitiger Schonung der fragilen Hohlfasern ermöglicht werden.

Gemäß einer bevorzugten Ausführungsform kann eine Größe der Querschnittsfläche der Vielzahl an Öffnungen mit einem zunehmenden Abstand der jeweiligen Öffnung von dem zumindest einen Anschluss zunehmen, sodass eine gleichmäßige Durchströmung der Vielzahl an Öffnungen mit dem Fluid über den Umfang des zylindrischen Hohlkörpers ermöglicht wird.

In der Nähe des Anschlusses würde bei identisch dimensionierten Öffnungen mehr Fluid durch die Öffnung strömen als bei einer weiter stromabwärts gelegenen Öffnung. Durch die Vergrößerung der Querschnittsfläche der weiter weg gelegenen Öffnungen wird der Strömungswiderstand reduziert.

Auf diese Weise kann vorteilhaft eine gleichmäßige Einströmung durch die Öffnungen bewirkt werden.

Diese gleichmäßige Einströmung kann sich vorteilhaft auf die Umströmung des Faserbündels auswirken und somit auf die Effizienz des Filters/ der Filtervorrichtung.

Gemäß einer bevorzugten Ausführungsform kann sich die Vielzahl an Öffnungen in einer axialen Richtung des zylindrischen Hohlkörpers verteilen.

In Kombination mit der Verteilung der Öffnungen in Umfangsrichtung kann somit ein flächiges Öffnungsmuster bereitgestellt werden. Auf diese Weise kann zum einen eine gezielte Umströmung des Faserbündels und zum anderen eine gezielte Reduzierung der Strömungsgeschwindigkeit auf synergetische Weise ermöglicht werden.

Gemäß einer bevorzugten Ausführungsform kann der zumindest eine Anschluss in axialer Richtung der Filtervorrichtung gegenüber dem zylindrischen Hohlkörper angeordnet ist, sodass die Strömung des Fluids auf den zylindrischen Hohlkörper trifft.

Mit anderen Worten ausgedrückt, befindet sich der zumindest eine Anschluss auf gleicher Höhe wie der zylindrische Hohlkörper.

Auf diese Weise können unnötige Wege für das Fluid vermieden werden. Dies ermöglicht ein kompaktes Design der Vorrichtung. Weiterhin werden unnötige Strömungsverluste reduziert, die einen effizienten Betrieb des Filters begünstigen.

Gemäß einer bevorzugten Ausführungsform kann die Querschnittsfläche der zumindest einen Öffnung eine oder mehr der folgenden Formen umfassen: Kreisform, ovale Form, Langlochform, Rechteckform und Quadratform.

Je nach gewünschter Filterleistung und umgebendem System (z. B. extrakorporale Blutbehandlungsmaschine) können unterschiedliche Formen für die Öffnung besser oder schlechter sein. Somit kann der Filter flexibel auf die gewünschten strömungstechnischen Anforderungen ausgelegt und dann entsprechend die Form gewählt werden. In Kombination mit einem sich weitendenden Querschnitt in radialer Richtung kann so auf synergetische Weise eine optimierte Durchströmung und Verteilung des Fluids gewährleistet werden.

Gemäß einer bevorzugten Ausführungsform kann der zylindrische Hohlköper als Material Kunststoff umfassen.

Kunststoff zeichnet sich durch günstige Bereitstellungskosten aus. Weiterhin eignet sich Kunststoff speziell für medizinische Anwendungen. Die Formgebung kann auf effiziente Weise über ein Spritzgusswerkzeug und einen entsprechenden Spritzguss erfolgen. Der Kunststoff kann beispielsweise Polypropylen oder Polyethylen umfassen.

Gemäß einer bevorzugten Ausführungsform kann der zylindrische Hohlkörper über ein Gießverfahren stoff- und/oder formschlüssig mit dem Gehäuse verbunden sein.

Die stoffschlüssige Verbindung über ein Gießverfahren wird bereits zur Anbindung des Faserbündels innerhalb des Gehäuses eingesetzt. Durch eine entsprechende Schnittstellengestaltung des zylindrischen Hohlkörpers kann dieser im gleichen Gießverfahren eingegossen werden. Hierzu wird der zylindrische Hohlkörper innerhalb des Gehäuses angeordnet und beispielsweise Polyurethan eingegossen. Diese Anbindung zeichnet sich durch niedrige Kosten aus.

Gemäß einer bevorzugten Ausführungsform kann die medizinische Filtervorrichtung weiter einen zweiten Anschluss umfassen, welcher an dem Gehäuse angeordnet ist und welcher vorgesehen ist, das Fluid aus dem Gehäuse ausströmen zu lassen. Der zumindest eine Anschluss ist somit bevorzugt ein erster Anschluss, welcher an dem Gehäuse angeordnet ist und welcher vorgesehen ist, das Fluid in das Gehäuse einströmen zu lassen.

Der zweite Anschluss kann identisch zum ersten Anschluss gestaltet sein. Der zweite Anschluss kann verschieden zum ersten Anschluss gestaltet sein. Der zweite Anschluss kann einen Kreislauf für das Fluid ermöglichen. Über den zweiten Anschluss kann das Fluid ausströmen. Im Falle einer Trocknungsluft kann der zweite Anschluss mit der Umgebung verbunden sein, sodass die Trocknungsluft einfach entweichen kann. Im Falle einer Dialysierflüssigkeit als Fluid kann der zweite Anschluss mit einem Abwasserbehälter verbunden sein oder mit einer Entsorgungsschnittstelle, so dass verbrauchte Dialysierflüssigkeit bzw. Dialysat über den zweiten Anschluss in den Abwasserbehälter oder die Entsorgungsschnittstelle abgeführt werden kann.

Gemäß einer bevorzugten Ausführungsform kann die medizinische Filtervorrichtung weiter eine Einlassschnittstelle für eine Ausgangsflüssigkeit, beispielsweise Blut, und eine Auslassschnittstelle für die gefilterte Ausgangsflüssigkeit (das gefilterte Blut) umfassen.

Die Eingangsschnittstelle und die Ausgangsschnittstelle können vorzugsweise mit einem extrakorporalen Blutkreislauf, insbesondere einem Schlauchsystem, einer extrakorporalen Blutbehandlungsvorrichtung verbunden sein. Der extrakorporale Blutkreislauf kann vorgesehen bzw. eingerichtet sein, an einen Menschen angeschlossen zu werden.

Gemäß einer bevorzugten Ausführungsform kann die Ausgangsflüssigkeit eines der folgenden umfassen: Blut, Wasser, Permeat, Dialysierflüssigkeit, Substitutionsflüssigkeit.

An dieser Stelle sei darauf hingewiesen, dass die medizinische Filtervorrichtung zum Filtern von Blut und eben auch Wasser/ Permeat/ Dialysierflüssigkeit/ Substitutionsflüssigkeit eingesetzt werden kann. Dies wirkt sich positiv auf die Flexibilität der Verwendung der medizinischen Filtervorrichtung aus.

Gemäß einer bevorzugten Ausführungsform kann das Fluid eines der folgenden umfassen: Wasser, Permeat, Dialysierflüssigkeit, kalte Luft und erhitzte Trocknungsluft.

Die kalte Luft dient dabei im Herstellungsprozess dem Ausblasen des Wassers vor dem Trocknen. Weiterhin kann die kalte Luft bei der Mikrowellentrocknung der medizinischen Filtervorrichtung Anwendung finden.

Ein weiterer Aspekt der vorliegenden Offenbarung betrifft eine extrakorporale Blutbehandlungsvorrichtung mit einer medizinischen Filtervorrichtung wie voranstehend beschrieben.

Die medizinische Filtereinrichtung kann dabei mehrfach, das heißt an mehreren Stellen, in der extrakorporalen Blutbehandlungsvorrichtung zum Einsatz kommen, beispielsweise zur Filterung von Blut als Dialysator und zur Filterung von Wasser/ Permeat/ Dialysierflüssigkeit/ Substitutionsflüssigkeit als Ultrafilter.

Die vorliegende Offenbarung wird nachfolgend mit Hilfe von Figuren erläutert. Es zeigen:
- Figur 1: eine Schnittansicht einer offenbarungsgemäßen medizinischen Filtervorrichtung,
- Figur 2: eine weitere Schnittansicht einer offenbarungsgemäßen medizinischen Filtervorrichtung,
- Figur 3: eine Detailansicht einer Schnittansicht einer offenbarungsgemäßen medizinischen Filtervorrichtung,
- Figur 4: eine isometrische Ansicht einer offenbarungsgemäßen medizinischen Filtervorrichtung,
- Figur 5: eine weitere Schnittansicht einer offenbarungsgemäßen medizinischen Filtervorrichtung,
- Figur 6: eine weitere Schnittansicht einer offenbarungsgemäßen medizinischen Filtervorrichtung, und
- Figur 7: eine offenbarungsgemäße extrakorporale Blutbehandlungsvorrichtung.

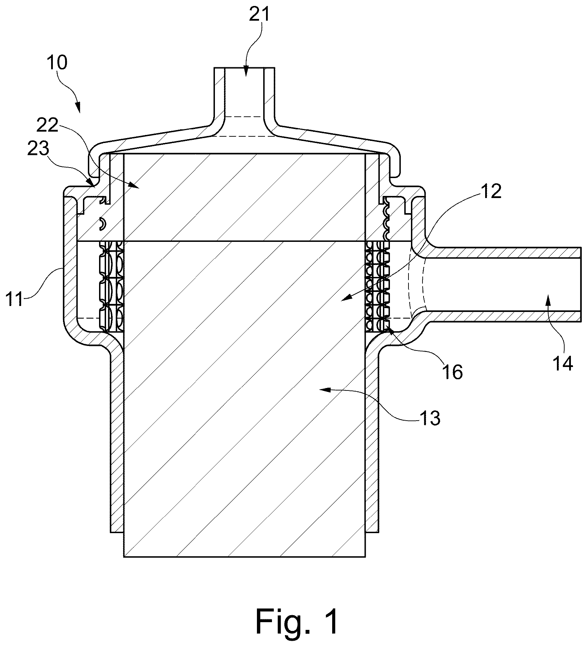

Figur 1 zeigt eine medizinische Filtervorrichtung 10 in einer Schnittdarstellung, wobei vorliegend lediglich die obere Hälfte der medizinischen Filtervorrichtung 10 dargestellt wird. Die untere Hälfte kann identisch zur oberen Hälfte aufgebaut sein. Die medizinische Filtervorrichtung 10 umfasst ein zylindrisches Gehäuse 11. Das Gehäuse 11 umfasst als Material einen Kunststoff. Innerhalb des Gehäuses 11 ist ein Faserbündel 13, das aus einer Vielzahl an Hohlfasern 12 gebildet ist, angeordnet. Das Faserbündel 13 ist über einen Polyurethangussbereich 22 mit dem Gehäuse 11 verbunden. Das Gehäuse 11 ist mehrteilig aufgebaut. Das Gehäuse 11 weist vorliegend ein Gehäusezwischenelement 23 auf. Die Filtervorrichtung 10 weist einen zylindrischen Hohlkörper 16 auf. Der zylindrische Hohlkörper 16 ist innerhalb des Gehäuse 11 angeordnet und ebenfalls über den Polyurethangussbereich 22 fixiert. Die Filtervorrichtung 10 weist einen Anschluss 14 für ein Fluid auf. Das Fluid ist vorliegend beispielsweise ein Wasser oder eine Dialysierflüssigkeit oder Heißluft. Die Filtervorrichtung 10 weist weiter eine Einlassschnittstelle 21 auf, über die eine Ausgangsflüssigkeit, beispielsweise Blut, in das Faserbündel 13, insbesondere in das Innere der Hohlfasern 12, strömt. Die Filtervorrichtung 10 weist weiterhin eine Auslassschnittstelle (nicht gezeigt) für die gefilterte Ausgangsflüssigkeit auf. Die Filtervorrichtung 10 weist weiterhin einen zweiten Anschluss (nicht gezeigt) für den Auslass des Fluids auf. Durch den Anschluss 14 strömt das Fluid und wird über den zylindrischen Hohlkörper 16 abgebremst und gleichmäßig um das Faserbündel 13 verteilt. Der Hohlkörper 16 weist hierzu Öffnungen 19 auf, die gleichmäßig über die Mantelfläche 18 in Umfangsrichtung und axialer Richtung verteilt sind.

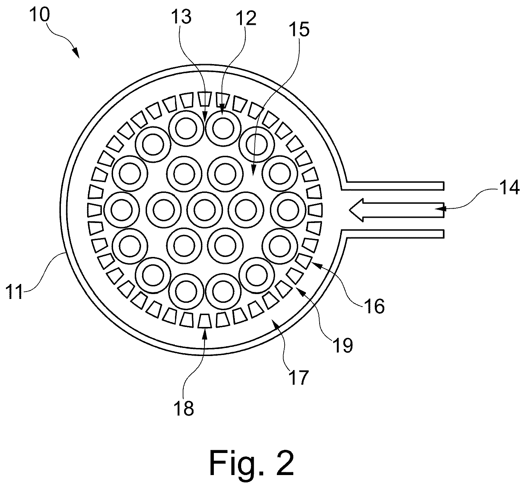

Figur 2 zeigt eine weitere Schnittansicht einer offenbarungsgemäßen medizinischen Filtervorrichtung 10. Hierbei sieht man, dass das Faserbündel 13 aus einzelnen Hohlfasern 12 besteht. Zwischen den Hohlfasern 12 sind Zwischenräume 15 vorhanden, durch die das Fluid strömen kann. Durch Diffusion durch die Wände der Hohlfasern 12 kann so das Blut gereinigt werden. Der zylindrische Hohlkörper 16 ist so innerhalb des Gehäuse 11 angeordnet, dass sich ein Ringkanal 17 zwischen dem Gehäuse 11 und dem zylindrischen Hohlkörper 16 bildet.

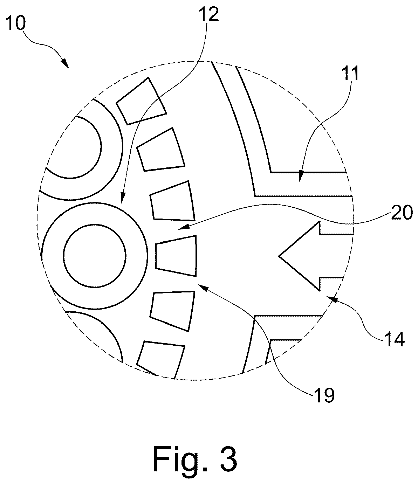

Figur 3 zeigt eine Detailansicht zu Figur 2. Hier ist deutlich zu erkennen, dass sich die Querschnittsfläche 20 der Öffnungen 20 radial von außen nach innen gesehen, vergrößert. Diese Vergrößerung führt zu einem Abbremsen der Strömung und bewirkt eine bessere Verteilung des Fluids sowie einen Schutz der fragilen Hohlfasern 12.

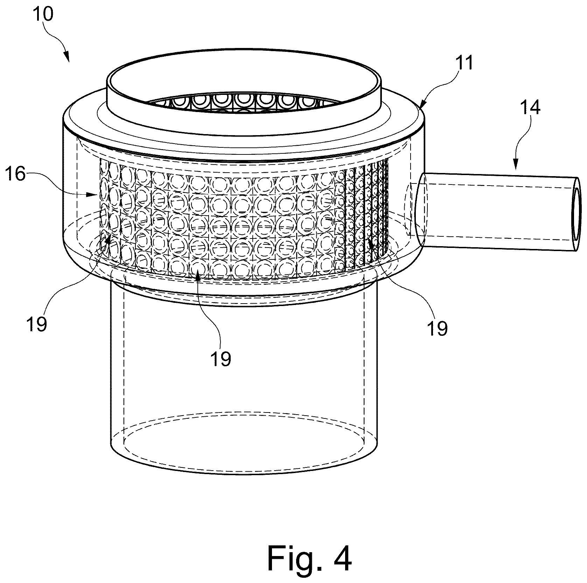

Figur 4 zeigt eine isometrische Ansicht einer medizinischen Filtervorrichtung 10. Die Öffnungen 19 werden mit zunehmenden Abstand vom Anschluss 14 größer. Dadurch kann eine bessere Verteilung des Fluids ermöglicht werden.

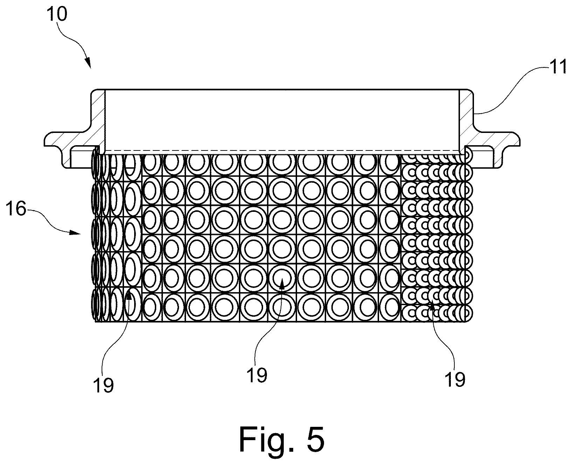

Figur 5 zeigt eine weitere Schnittansicht einer medizinischen Filtervorrichtung 10. Hier ist ebenfalls zu erkennen, dass sich die Öffnungen 19 mit zunehmenden Abstand vom Anschluss 14 vergrößern, um eine gleichmäßigere Verteilung des Fluids zu ermöglichen.

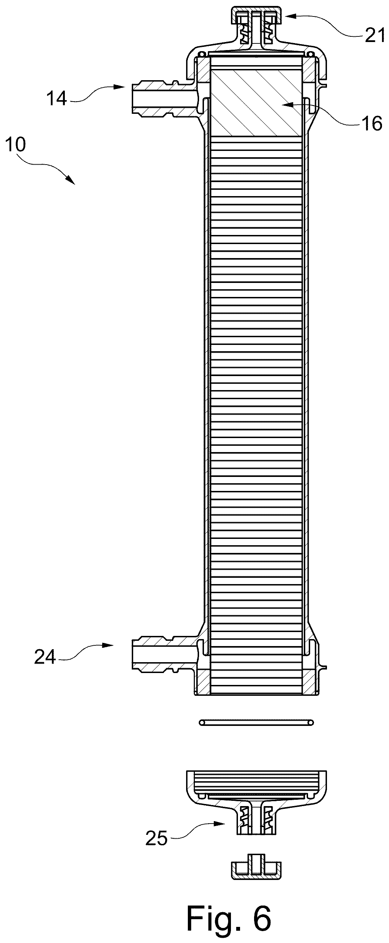

Figur 6 zeigt eine weitere Schnittansicht einer offenbarungsgemäßen medizinischen Filtervorrichtung 10. Im Gegensatz zu Figur 1 sind vorliegend weiter der zweite Anschluss 24 für den Auslass des Fluids sowie die Auslassschnittstelle 25 für die gefilterte Ausgangsflüssigkeit zu sehen.

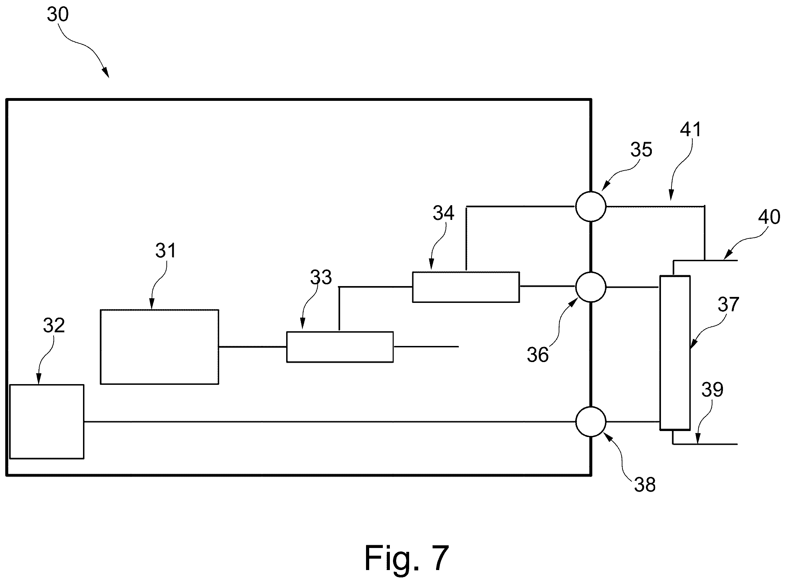

Figur 7 zeigt eine vereinfachte Darstellung einer offenbarungsgemäßen extrakorporalen Blutbehandlungsvorrichtung 30. Die extrakorporale Blutbehandlungsvorrichtung 30 umfasst eine Quelle 31 für ein Fluid. Die extrakorporale Blutbehandlungsvorrichtung 30 umfasst zwei Filtervorrichtungen 33, 34, über die das Fluid gefiltert wird. Die Filtervorrichtungen 33, 34 fungieren als Ultrafilter. Das über die Filtervorrichtung 33 gefilterte Fluid wird dann als Dialysierflüssigkeit verwendet. Das über beide Filtervorrichtungen 33, 34 gefilterte Fluid wird über eine Schnittstelle 35 als Substituat weiterverwendet und in eine Substituatleitung 41 geleitet. Das einmal gefilterte Fluid kann beispielsweise über die Schnittstelle 36 einer Filtervorrichtung 37 zugeführt werden, die als Dialysator fungiert und mit einer Blutzuflussleitung 39 und einer Blutabflussleitung 40 verbunden ist. Über eine Schnittstellstelle 38 kann das verwendete Fluid, insbesondere Dialysat, wiederum einem Entsorgungsbehälter 32 zugeführt werden. Die Verbindungslinien stellen Leitungen dar. Aus Übersichtsgründen wurden keine Pumpen, Heizelemente, Ventile, Steuerungen und dergleichen dargestellt. Weiterhin wurden aus Übersichtsgründen im Wesentlichen lediglich die für die vorliegende Offenbarung relevanten Elemente der extrakorporalen Blutbehandlungsvorrichtung 30 dargestellt.

### Bezugszeichenliste

- 10: medizinische Filtervorrichtung
- 11: Gehäuse
- 12: Hohlfaser
- 13: Faserbündel
- 14: Anschluss
- 15: Zwischenraum
- 16: zylindrischer Hohlkörper
- 17: Ringkanal
- 18: Mantelfläche
- 19: Öffnung
- 20: Querschnittsfläche
- 21: Einlassschnittstelle
- 22: PU Gussbereich
- 23: Gehäusezwischenelement
- 24: zweiter Anschluss
- 25: Auslassschnittstelle

- 30: Blutbehandlungsvorrichtung
- 31: Quelle
- 32: Entsorgungsbehälter
- 33: Filtervorrichtung, Ultrafilter
- 34: Filtervorrichtung, Ultrafilter
- 35: Schnittstelle
- 36: Schnittstelle
- 37: Filtervorrichtung, Dialysator
- 38: Schnittstelle
- 39: Blutzuflussleitung
- 40: Blutabflussleitung

## Patentansprüche

1. Medizinische Filtervorrichtung (10), vorzugsweise Dialysator oder Ultrafilter, umfassend
ein Gehäuse (11),
eine Vielzahl von Hohlfasern (12), welche ein Faserbündel (13) bilden, das in dem Gehäuse (11) angeordnet ist,
zumindest einen Anschluss (14), welcher an dem Gehäuse (11) angeordnet ist und welcher vorgesehen ist, ein Fluid, vorzugsweise Dialysierflüssigkeit, in das Gehäuse (11), insbesondere in einen Zwischenraum (15) zwischen der Vielzahl von Hohlfasern (12), einströmen zu lassen,
einen zylindrischen Hohlkörper (16), insbesondere Diffusorring, der innerhalb des Gehäuses (11) derart angeordnet ist, dass sich ausgehend von dem zumindest einen Anschluss (14) ein Ringkanal (17) zwischen dem zylindrischen Hohlkörper (16) und dem Gehäuse (11) ausbildet,
wobei der zylindrische Hohlkörper (16) das Faserbündel (13) umfasst und dabei zentrisch in dem Gehäuse (11) anordnet,
wobei der zylindrische Hohlkörper (16) an seiner Mantelfläche (18) zumindest eine Öffnung (19) aufweist, die eine Querschnittsfläche (20) aufweist,
wobei die zumindest eine Öffnung (19) durchgängig ist,
wobei sich die Querschnittsfläche (20) zumindest teilweise in radialer Richtung von außen nach innen vergrößert, so dass sich eine Strömung des Fluids beim Durchströmen der zumindest einen Öffnung (19) verlangsamt.

2. Medizinische Filtervorrichtung (10) nach Anspruch 1, wobei die zumindest eine Öffnung (19) eine Vielzahl an Öffnungen (19) umfasst, die über einen Umfang des zylindrischen Hohlkörpers (16) verteilt sind.

3. Medizinische Filtervorrichtung (10) nach Anspruch 2, wobei eine Größe der Querschnittsfläche (20) der Vielzahl an Öffnungen (19) mit einem zunehmenden Abstand der jeweiligen Öffnung (19) von dem zumindest einen Anschluss (14) zunimmt, sodass eine gleichmäßige Durchströmung der Vielzahl an Öffnungen (19) mit dem Fluid über den Umfang des zylindrischen Hohlkörpers (16) ermöglicht wird.

4. Medizinische Filtervorrichtung (10) nach Anspruch 2 oder 3, wobei sich die Vielzahl an Öffnungen (19) in einer axialen Richtung des zylindrischen Hohlkörpers (16) verteilt.

5. Medizinische Filtervorrichtung (10) nach einem der vorherigen Ansprüche, wobei der zumindest eine Anschluss (14) in axialer Richtung der Vorrichtung (10) gegenüber dem zylindrischen Hohlkörper (16) angeordnet ist, sodass die Strömung des Fluids auf den zylindrischen Hohlkörper (16) trifft.

6. Medizinische Filtervorrichtung (10) nach einem der vorherigen Ansprüche, wobei die Querschnittsfläche (20) der zumindest einen Öffnung (19) eine oder mehr der folgenden Formen umfasst: Kreisform, ovale Form, Langlochform, Rechteckform und Quadratform.

7. Medizinische Filtervorrichtung (10) nach einem der vorherigen Ansprüche, wobei der zylindrische Hohlköper (16) als Material Kunststoff umfasst.

8. Medizinische Filtervorrichtung (10) nach einem der vorherigen Ansprüche, wobei der zylindrische Hohlkörper (16) über ein Gießverfahren stoff- und/oder formschlüssig mit dem Gehäuse (11) verbunden ist, insbesondere in das Gehäuse (11) eingegossen ist.

9. Medizinische Filtervorrichtung (10) nach einem der vorherigen Ansprüche, weiter umfassend einen zweiten Anschluss (24), welcher an dem Gehäuse (11) angeordnet ist und welcher vorgesehen ist, das Fluid aus dem Gehäuse (11) ausströmen zu lassen.

10. Medizinische Filtervorrichtung (10) nach einem der vorherigen Ansprüche, weiter umfassend eine Einlassschnittstelle (21)für eine Ausgangsflüssigkeit und eine Auslassschnittstelle (25) für die gefilterte Ausgangsflüssigkeit.

11. Medizinische Filtervorrichtung (10) nach einem der vorherigen Ansprüche, wobei die Ausgangsflüssigkeit eines der folgenden umfasst: Blut, Wasser, Permeat, Dialysierflüssigkeit, Substitutionsflüssigkeit, kalte Luft, erhitzte Trocknungsluft.

12. Medizinische Filtervorrichtung (10) nach einem der vorherigen Ansprüche, wobei das Fluid eines der folgenden umfasst: Wasser, Permeat, Dialysierflüssigkeit, Substitutionsflüssigkeit und erhitzte Trocknungsluft.

13. Extrakorporale Blutbehandlungsvorrichtung (30) mit einer medizinischen Filtervorrichtung (10) nach einem der Ansprüche 1 bis 12.
